# EUROPÄISCHE PATENTSCHRIFT

(11) **EP 1 372 826 B1**
(45) Veröffentlichungstag und Bekanntmachung des Hinweises auf die Patenterteilung: **02.03.2005**
(21) Anmeldenummer: 02716629.7
(22) Anmeldetag: 27.02.2002
(51) Int. Cl.: B01D 53/86

(54) **VERFAHREN UND VORRICHTUNG ZUR BEURTEILUNG DER FUNKTIONSFÄHIGKEIT EINER EINRICHTUNG ZUR REDUZIERUNG DES OZONGEHALTES VON LUFT**
METHOD AND DEVICE FOR ASSESSING THE OPERABILITY OF A DEVICE FOR REDUCING THE OZONE CONTENT IN THE AIR
PROCEDE ET DISPOSITIF POUR EVALUER L'APTITUDE AU FONCTIONNEMENT D'UN DISPOSITIF DE REDUCTION DE LA TENEUR EN OZONE DE L'AIR

(30) Priorität: 28.03.2001 DE 10115219
(43) Veröffentlichungstag der Anmeldung: 02.01.2004
(73) Patentinhaber: ROBERT BOSCH GMBH, 70442 Stuttgart (DE)
(72) Erfinder: KLEE, Peter, 75438 Knittlingen (DE); DAMBACH, Dieter-Andreas, 70825 Korntal-Muenchingen (DE); KNIRSCH, Matthias, 71701 Schwieberdingen (DE)
(86) Internationale Anmeldenummer: PCT/DE2002/000723
(87) Internationale Veröffentlichungsnummer: WO 2002/076584

(56) Entgegenhaltungen:
- EP-A- 0 431 648
- EP-A- 0 467 526
- EP-A- 0 501 003
- EP-A- 1 153 647
- DE-A- 3 938 592

## Beschreibung

### Stand der Technik

Die Erfindung betrifft die Diagnose einer DOR-Einrichtung, das heißt einer Einrichtung zur direkten Ozon Reduzierung. Reduziert werden soll der Ozongehalt der die Einrichtung umgebenden oder durchströmenden Luft.

Aus der US 5,97,831A ist bereits ein katalytisch beschichteter Kühler für ein Fahrzeug bekannt geworden, mit dem Ozon (03) in der Umgebung in Sauerstoff (02) konvertiert werden kann. In der amerikanischen Abgasgesetzgebung (CARB) ist der Ausstoß an Non-Methane Organic Gases (NMOG) in Form eines Flottendurchschnittswerts für jeden Fahrzeughersteller limitiert. Es besteht grundsätzlich die Möglichkeit, NMOG-Credit zu sammeln. Damit kann der Flottendurchschnitt bezüglich in NMOG abgesenkt werden. Damit hat der Fahrzeughersteller auch die Möglichkeit, den Abstand zu den Niedrigst-Emissions-Grenzwerten (SULEV) zu vergrößern. Unter anderem bietet auch die katalytische Beschichtung des Fahrzeugkühlers zum Zweck der Minderung des Ozongehalts der Umgebungsluft die Möglichkeit, in NMOG-Credits zu sammeln. Die genannte katalytische Beschichtung wird von den US-Behörden als Direct Ozon Reduction (DOR) bezeichnet. Die Bezeichnung DOR soll auch im Folgenden, allerdings nur stellvertretend für das katalytische Prinzip, verwendet werden. Wie alle Fahrzeugeinrichtung zur Emissionsminderung muss auch dieses System im Rahmen der gesetzlichen On-Board-Diagnose-Forderungen auf seine Funktionstüchtigkeit überwacht werden.

Aus der DE 39 38 592 A1 ist eine Vorrichtung zur Beseitigung des Ozongehalts von Raumluft bekannt geworden, bei der ein erster Ozonsensor den Ozongehalt der ungereinigten und ein zweiter Ozonsensor den der gereinigten Luft erfasst. Durch einen Vergleich der beiden Sensorsignale kann auf die Funktionsfähigkeit der DOR-Einrichtung geschlossen werden.

Vor diesem Hintergrund besteht die Aufgabe der Erfindung in der Angabe einer Vorrichtung zur Beurteilung der Funktionsfähigkeit einer DOR-Einrichtung, die kostengünstig zu realisieren ist.

Die Aufgabe wird durch die im unabhängigen Anspruch angegebenen Merkmale gelöst.

### Vorteile der Erfindung

Die erfindungsgemäße Vorrichtung zur Beurteilung der Funktionsfähigkeit einer in einem Kraftfahrzeug angeordneten DOR-Einrichtung zur Reduzierung des Ozongehalts von Luft sieht einen Sensor zum Erfassen des Ozongehalts vor, der in Strömungsrichtung hinter der DOR-Einrichtung angeordnet ist. Vorgesehen ist eine elektronische Einrichtung, die bei einer ersten Geschwindigkeit des Kraftfahrzeugs einem ersten Ozongehalt und bei einer zweiten Geschwindigkeit einen zweiten Ozongehalt erfasst. Die elektronische Einrichtung ermittelt eine Differenz zwischen dem ersten und zweiten Ozongehalt und beurteilt anhand einer hinterlegten Kennlinie die Funktionsfähigkeit der DOR-Einrichtung.

Die erfindungsgemäße Vorrichtung weist den wesentlichen Vorteil auf, dass nur ein Sensor zum Erfassen des Ozongehalts der Luft erforderlich ist.

Vorteilhafte Weiterbildungen und Ausgestaltungen der erfindungsgemäßen Vorrichtung ergeben sich aus abhängigen Ansprüchen.

Eine Ausgestaltung sieht vor, dass die erste und zweite Geschwindigkeit in vorgegebenen Geschwindigkeitsbereichen liegen.

Eine Ausgestaltung sieht vor, dass die erste Geschwindigkeit des Kraftfahrzeugs der Geschwindigkeit null entspricht.

Eine Ausgestaltung sieht vor, dass die Zeitspanne zwischen der Erfassen des ersten und zweiten Ozongehalts auf einen vorgegebenen maximalen Wert begrenzt ist.

Eine Ausgestaltung sieht vor, das zusätzlich ein Schwellenwert für die Motorkühlmittel-Temperatur vorgesehen ist.

Eine Ausgestaltung sieht vor, dass die DOR-Einrichtung einen mit katalytisch wirkenden Oberflächenbeschichtungen ausgestatteten Wärmetauscher aufweist.

Weitere vorteilhafte Ausgestaltungen und Weiterbildungen der erfindungsgemäßen Vorrichtung ergeben sich aus weiteren abhängigen Ansprüchen und aus der folgenden Beschreibung.

### Zeichnung

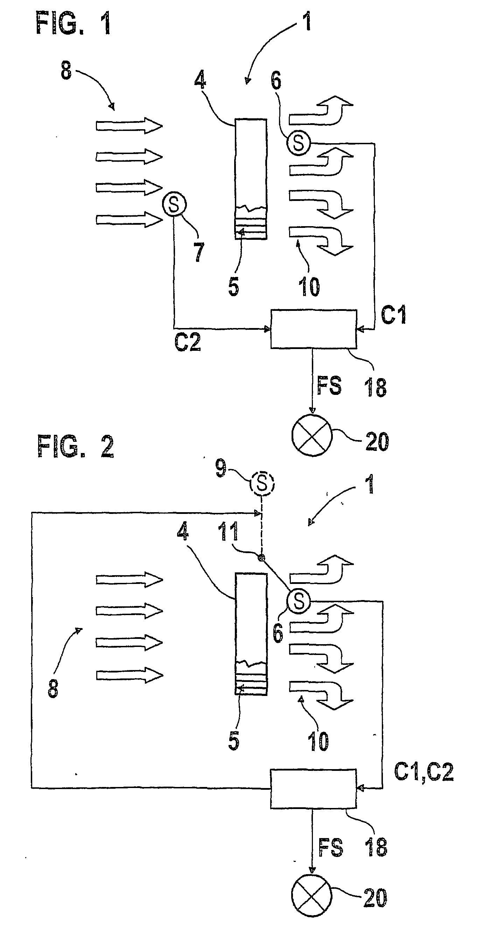

Im Einzelnen zeigt Figur 1 ein Ausführungsbeispiel einer bekannten Vorrichtung mit zwei räumlich getrennt angeordneten Mitteln 6 und 7 zur Erfassung der Ozongehalte.
Figur 2 zeigt eine weitere nicht erfindungsgemäße Ausführungsform mit nur einem Mittel zur Erfassung eines Ozongehalts.
Figur 3 stellt eine Ausführungsform der Erfindung mit nur einem Mittel 6 zur Erfassung eines Ozongehalts dar.
Figur 4 zeigt den Verlauf der Ozonumwandlungsrate OUR, die einem Maß für die Einwirkung E der DOR-Einrichtung auf die Luft entspricht, in Abhängigkeit von der Fahrzeug-, bzw. Raumgeschwindigkeit der Luft durch die DOR-Einrichtung.
Figur 5 zeigt ein Flußdiagramm als exemplarische Ausführungsform eines erfindungsgemäßen Verfahrens.

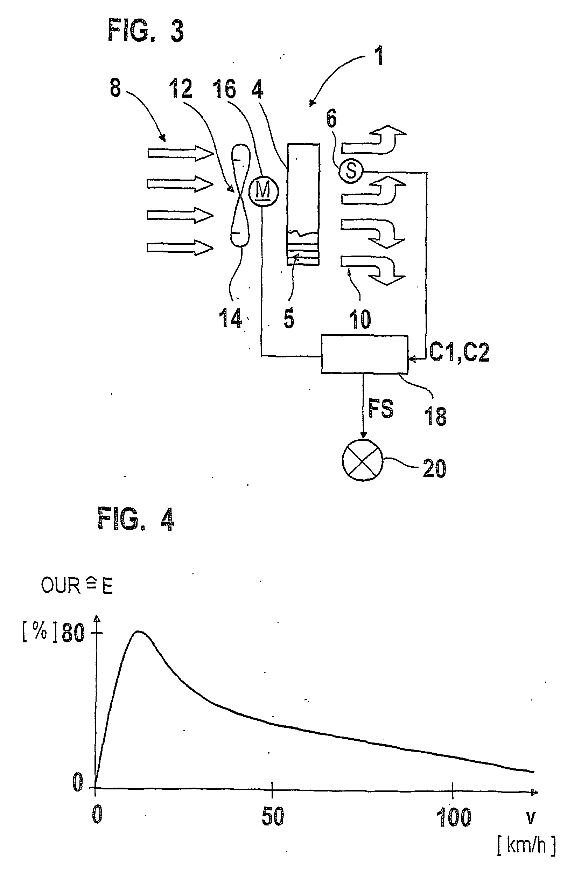

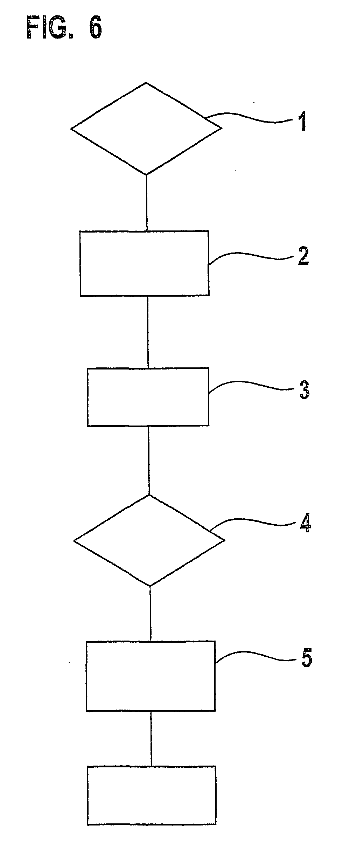

Das Flußdiagramm der Figur 6 ist insbesondere in Verbindung mit den Vorrichtungen nach Figur 2 und Figur 3 ausführbar.

### Beschreibung

Die Ziffer 1 in der Figur 1 bezeichnet die räumliche Anordnung einer DOR-Einrichtung 4 und weiterer Komponenten im Rahmen einer bekannten Ausführungsform. Die DOR-Einrichtung weist katalytisch beschichtete Oberflächen 5 auf. Die Ziffer 6 bezeichnet ein erstes Mittel zur Erfassung eines Ozongehaltes, bspw. einen Ozon-Sensor. Es erfaßt den Ozongehalt C1 der Luft nach der Einwirkung der DOR-Einrichtung. Diese Luft wird durch die mit der Ziffer 10 bezeichneten Pfeile repräsentiert. Entsprechend bezeichnet die Ziffer 7 ein zweites Mittel zur Erfassung des Ozongehaltes der Luft. Dieses Mittel erfaßt den Ozongehalt C2 der Luft bei einer zweiten, von der ersten abweichenden Einwirkung der DOR-Einrichtung. Im Beispiel der Figur 1 wird diese Luft durch die mit der Ziffer 8 bezeichneten Pfeile vor der DOR-Einrichtung repräsentiert und die zweite Einwirkung ist gleich 0. Entsprechend repräsentieren die mit der Ziffer 10 bezeichneten Pfeile die Luft bei einer zweiten Einwirkung der DOR-Einrichtung. Im Beispiel der Figur 1 entspricht diese Luft der Luft nach der Einwirkung der DOR-Einrichtung 4.

Die Signale der beiden Mittel 6 und 7, die für den ersten (C1) und zweiten (C2) Ozongehalt stehen, werden einer elektrischen Einrichtung 18 zur Aufbereitung und Auswertung zugeführt. Die Auswertung schließt das Bilden eines Vergleichsergebnisses VE aus einem Vergleich des ersten Ozongehalts C1 mit dem zweiten Ozongehalt C2 und eine Beurteilung der Funktionsfähigkeit der DOR-Einrichtung auf der Basis des Vergleichsergebnisses ein. Wenn der Vergleich eine nicht ausreichende Funktionsfähigkeit ergibt, erfolgt die Anzeige oder Speicherung einer entsprechenden Fehlermeldung. Die Anzeige kann beispielsweise nach statistischer Absicherung durch eine Fehlerlampe 20 im Gesichtsfeld des Fahrers eines mit einer DOR-Einrichtung ausgerüsteten Kraftfahrzeuges erfolgen.

Figur 2 zeigt eine nicht erfindungsgemäße Ausführungsform mit nur einem Mittel 6 zur Erfassung eines Ozongehalts. Die Ziffer 9 in der Figur 2 bezeichnet das Mittel 6 in einer zweiten Position (gestrichelte Darstellung). In der mit einer durchgezeichneten Linie dargestellten Position erfaßt das Mittel 6 den Ozongehalt nach der Einwirkung der DOR-Einrichtung, d. h. bei einer ersten Einwirkung E1 der DOR-Einrichtung. In der gestrichelt gezeichneten Position 9 erfaßt das Mittel 6 den Ozongehalt außerhalb der aus der DOR-Einrichtung ausströmenden Luft und damit bei einer zweiten (E2), von der ersten (E1) abweichenden Einwirkung E der DOR-Einrichtung. Die Positionsveränderung kann beispielsweise elektromechanisch erfolgen und durch die elektronische Einrichtung 18 gesteuert werden. Auf diese Weise erfolgt das Erfassen des ersten Ozongehaltes C1 an einem ersten Ort räumlich getrennt von dem Erfassen des zweiten Ozongehaltes C2 an einem zweiten Ort mit Hilfe einer Positionsveränderung eines einzigen Mittels 6. Bei einem katalytisch beschichteten Fahrzeugkühler kann beispielsweise der Ozon-Sensor mittels einer geeigneten Vorrichtung bewegt werden, so daß er zum einen die Roh-Umgebungsluft vor Durchströmen des Fahrzeugkühlers, zum anderen die Umgebungsluft nach Durchströmen des Fahrzeugkühlers messen kann.

Figur 3 stellt eine Ausführungsform der Erfindung mit nur einem Mittel 6 zur Erfassung eines Ozongehaltes dar. Hier erfolgt das Erfassen der Ozongehalte bei verschiedenen Einwirkungen der DOR-Einrichtung nicht räumlich getrennt, sondern zeitlich getrennt. Das Erfassen des ersten Ozongehalts (C1) findet zu einem ersten Zeitpunkt T1 am gleichen Ort statt, wie das Erfassen des zweiten Ozongehaltes (C2) zu einem zweiten Zeitpunkt T2.

Die Ozongehalte zu beiden Zeitpunkten unterscheiden sich bei funktionsfähiger DOR-Einrichtung dann, wenn zwischen beiden Zeitpunkten die Einwirkung E der DOR-Einrichtung auf die Luft geändert wurde oder sich geändert hat.

Zur Änderung der Einwirkung E eignet sich beispielsweise eine Änderung der Raumgeschwindigkeit, mit der die Luft durch die DOR-Einrichtung strömt. Unter Raumgeschwindigkeit wird hier das pro Zeiteinheit durch die DOR-Einrichtung strömende Luftvolumen verstanden, das bei einem Kraftfahrzeug beispielsweise mit der Fahrzeuggeschwindigkeit v korreliert. Bei einem Fahrzeugkühler kann bspw. bei fester Position eines Ozon-Sensors eine Ozonmessung bei verschiedenen Fahrzeug- und Motor-Betriebsbedingungen erfolgen: In Abhängigkeit von verschiedenen Fahrzeug- und Motor-Betriebsbedingungen befindet sich an der Position des Ozon-Sensors entweder Roh-Umgebungsluft, oder durch den Fahrzeugkühler geströmte Umgebungsluft.

Alternativ kann bei fester Position des Sensors die Kühlerdurchströmung temporär verhindert werden. Durch temporäre Verhinderung des Durchströmens des Fahrzeugkühlers z.B. durch eine Kühlerjalousie oder durch eine umschaltbare Zuführung von Umgebungsluft (Kühlerumgehung) kann sichergestellt werden, daß sich am Ozon-Sensor einmal Roh-Umgebungsluft und einmal konvertierte Luft befindet.

Eine einfache Ausführungsform benutzt eine Vergleichsmessung zwischen Roh-Umgebungsluft und Umgebungsluft nach Durchströmen des katalytisch beschichteten Fahrzeugkühlers. Zur Diagnose der Konvertierungsfähigkeit eines DOR Systems mittels einem Ozon-Sensor ist der Ozon-Sensor derart in der Nähe des Fahrzeugkühlers anzubringen, dass die durch den Fahrzeugkühler strömende Luft gemessen werden kann.

Nur die Ausführungsform, bei der die Einwirkung durch eine Änderung der Fahrzeuggeschwindigkeit erzeugt wird, ist erfindungsgemäß.

Für alle drei Meß-Prinzipien wird die Art und Weise der Durchströmung des Fahrzeugkühlers in verschiedenen Fahrzeug- und Motor-Betriebspunkten vorteilhafterweise im Rahmen von Grundsatzuntersuchungen gemessen und die Ergebnisse werden in Kennfeldern des Fahrzeugsteuergerätes abgelegt, so daß für die Ozon-Messung während des Fahrzeug-Serienbetriebs geeignete Referenzwerte greifbar sind. Dies betrifft insbesondere die Messung der Luft nach Durchströmen des Kühlers, welche von verschiedenen Bedingungen abhängt, wie z.B. Motorkühlmittel-Temperatur, Motor-Drehzahl, Motorlüfter-Drehzahl, Fahrzeuggeschwindigkeit etc.

Neben anderen können insbesondere die folgenden Effekte zur Vergleichsmessung herangezogen werden:
- abnehmende Ozon-Konvertierung bei zunehmender Raumgeschwindigkeit
- keine Ozon-Konvertierung ohne Durchströmen (Raumgeschwindigkeit = Null)

Beispiele für die Realisierung einer Diagnosefunktion mit einer Vorrichtung nach Fig. 3, obwohl nur die Fälle A) und B) efindungsgemäße Beispiele sind:
A) Auswertung der Differenz der O3-Konzentration bei stehendem und fahrendem Fahrzeug:
   Feste Anbringung des O3-Sensors hinter dem Kühler, also zwischen Kühler und Motor. Erste Messung des Ozongehalts der den Sensor umgebenden Luft, sobald folgende Randbedingungen für eine vorzugebende Zeitspanne (z.B. 30 sec.) erfüllt sind:
      - Motor betriebswarm (Kühler zeigt gute O3-Konvertierungsrate)
      - Fahrzeug steht (v=0)
      - Motor-Lüfterräder stehen still
   Zweite Messung erfolgt beim nächsten Anfahren, sobald die Fahrzeuggeschwindigkeit einen vorgegebenen Wert erreicht (z.B. 8 km/h)
   Falls die DOR-Einrichtung korrekt arbeitet, muß die Differenz der beiden gemessenen O3-Konzentrationswerte einen bestimmten, vorgegebenen Wert übersteigen.
B) Auswertung der Differenz der O3-Konzentration bei mind. 2 unterschiedlichen Fahrzeug-Geschwindigkeiten:
   Feste Anbringung des O3-Sensors hinter dem Kühler, also zwischen Kühler und Motor.
   Erste Messung des Ozongehalts der den Sensor umgebenden Luft, sobald folgende Randbedingungen erfüllt sind:
      - Motor betriebswarm
      - Fahrzeug fährt mit niedriger Geschwindigkeit innerhalb eines vorzugebenden Geschwindigkeitsbereiches (z.B. v= 6 ... 8 km/h)
      - Motor-Lüfterräder stehen still
   Die zweite Messung erfolgt, falls die Fahrzeug-Geschwindigkeit innerhalb einer vorzugebenden Zeitspanne nach der ersten Messung (z.B. 30 sec) einen vorgegebenen Wert erreicht (z.B. 70 km/h)
   Falls die DOR-Einrichtung korrekt arbeitet, muß die Differenz der beiden gemessenen O3-Konzentrationswerte einen bestimmten, vorgegebenen Wert übersteigen, da die O3-Konvertierungrate oberhalb ca. 5 km/h mit zunehmender Geschwindigkeit abnimmt. Falls beide Meßwerte dicht beieinander liegen, ist dies ein Indiz dafür, daß die DOR-Einrichtung nicht mehr einwandfrei arbeitet.
C) Auswertung der Differenz der O3-Konzentration bei stehendem Fahrzeug vor und nach Einschalten des Lüfters:
   Feste Anbringung des O3-Sensors in Strömungsrichtung hinter dem Kühlerlüfter, zwischen Kühler und Verbrennungsmotor. Erste Messung des Ozongehalts der den Sensor umgebenden Luft erfolgt, sobald folgende Randbedingungen für eine vorzugebende Zeitspanne (z.B. 30 sec.) erfüllt sind:
      - Motor betriebswarm (Kühler zeigt z.B. bei Tmot > 80°C gute O3-Konvertierungsrate)
      - Fahrzeug steht (v=0)
      - Motor-Lüfterräder stehen still

Dann Einschalten des Lüfters zu Diagnosezwecken und zweite Messung des Ozongehaltes. Der Lüfter erzeugt eine Durchströmung des Kühlers, wobei eine funktionierende DOR-Einrichtung den Ozongehalt der vom Lüfter zum 03-Sensor geförderten Luft reduziert.

Falls das DOR-System korrekt arbeitet, muß die Differenz der beiden gemessenen O3-Konzentrationswerte einen bestimmten, vorgegebenen Wert übersteigen.

Zur Erhöhung der Diagnose-Sicherheit kann eine statistische Auswertung mehrerer Vergleichsmessungen erfolgen, die zum endgültigen Diagnose-Ergebnis führt. Diese Auswertung kann sich auch über mehrere Fahrten erstrecken.

Figur 4 zeigt den Verlauf der Ozonumwandlungsrate OUR, die einem Maß für die Einwirkung E der DOR-Einrichtung auf die Luft entspricht, in Abhängigkeit von der Fahrzeuggeschwindigkeit, bzw. der Raumgeschwindigkeit der Luft durch die DOR-Einrichtung. Die in Praxis erzielbare Ozon-Konvertierungsrate hängt in geringem Maße von der Temperatur der Beschichtung oder von der absoluten Ozon-Konzentration, in hohem Maße von der Raumgeschwindigkeit der Umgebungsluft durch den Fahrzeugkühler ab.

Bei der in Figur 3 dargestellten Ausführungsform erfolgt die Erfassung der Ozongehalte C1, C2 beispielsweise bei verschiedenen Raumgeschwindigkeiten und damit verschiedener Einwirkung. In diesem Sinne stellt der Fahrzeugantrieb gewissermaßen ein Mittel 12 zur Veränderung der Einwirkung dar. Ergänzend oder alternativ zur Änderung der Raumgeschwindigkeit durch eine Änderung der Fahrzeuggeschwindigkeit kann die Raumgeschwindigkeit und damit die Einwirkung auch durch eine Änderung der Luftförderung eines Ventilators 14 erfolgen. Beispielsweise kann die Drehzahl eines den Ventilator antreibenden Elektromotors 16 durch die elektronische Einrichtung 18 entsprechend verstellt werden.

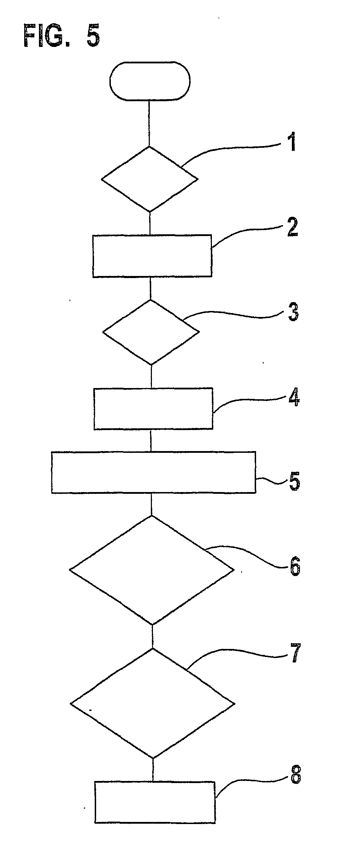

Figur 5 zeigt ein Flußdiagramm eines Verfahrens - bei dem nur im Fall von Einwirkungen verschiedener Fahrzeuggeschwindigkeiten gemessen wird - das als erfindungsgemäß erachtet wird. Nach dem Start des Verfahrens wird in Schritt 1 überprüft, ob die Einwirkung E des DOR-Systems einer ersten Einwirkung E1 entspricht, die zur Erfassung der ersten Konzentration C1 geeignet ist. Beispielsweise müssen die Sensoren betriebsbereit sein und die katalytischen Oberflächen sollten betriebswarm sein. Im Schritt 2 wird dann die Ozonkonzentration C1 bei der ersten Einwirkung E1 erfaßt. Wenn die Bedingungen zur Erfassung der zweiten Ozonkonzentration C2 erfüllt sind (Schritt 3) erfolgt im Schritt 4 die Erfassung des Sauerstoffgehaltes C2 bei der zweiten Einwirkung E2. Anschließend wird in Schritt 5 ein Vergleichsergebnis VE als Funktion der erfaßten Ozongehalt C1, C2 und gegebenenfalls weiterer Parameter wie der Temperatur zum Testzeitpunkt usw. gebildet. In den Schritten 6 und 7 wird das Vergleichsergebnis mit vorgegebenen Grenzen verglichen, die einen Gutbereich (Schritt 6) bzw. ein Schlechtbereich (Schritt 7) definieren. Wenn das Vergleichsergebnis einem Element aus dem Schlechtbereich entspricht, erfolgt die Ausgabe eines Fehlersignales im Schritt 8. Das Vergleichsergebnis liegt beispielsweise im Gutbereich, wenn die Differenz der beiden gemessenen Ozongehaltswerte einen bestimmten, vorgegebenen Wert übersteigt. Die Ausführungsform nach Figur 5 läßt sich in Verbindung mit den Vorrichtungen nach Figur 1, 2 oder 3 nutzen. In Verbindung mit Figur 2 umfaßt beispielsweise die Abfrage in Schritt 1 der Figur 5 die Prüfung, ob das Mittel zur Erfassung des Ozongehaltes in Position 6 oder in Position 9 ist. Dabei entspricht die Bedingung E = E1 der Position 6. Analog entspricht die Bedingung E = E2 der Position 9. In Verbindung mit Figur 3 entspricht die Bedingung E = E1 einem angetriebenen Lüfter 14 und die Bedingung E = E2 entspricht einem stillstehenden Lüfterrad. Analog kann E = E1 einem fahrenden Fahrzeug und E = E2 einem stillstehendem Fahrzeug oder einem mit niedrigerer Geschwindigkeit fahrenden Fahrzeug entsprechen. Nur dieser letzte Fall Kann als erfindungsgemäß angesehen werden.

Das Flußdiagramm der Figur 6 ist insbesondere in Verbindung mit den Vorrichtungen nach Figur 2 und Figur 3 ausführbar. Wieder wird in einem Schritt 1 geprüft, ob die Einwirkung E des DOR-Systems einer Einwirkung E1 entspricht, die zur Erfassung eines Ozongehaltes C1 geeignet ist. Wenn dies der Fall ist, erfolgt im Schritt 2 die Erfassung des Ozongehaltes C1 zum Zeitpunkt T = T1. Anschließend wird im Schritt 3 die Einwirkung E verändert. Möglichkeiten zur Veränderung der Einwirkung E auf die Luft am Sensorort sind: die Veränderung der Sensorpositionen, wie in Figur 2 dargestellt; die Veränderung der Raumgeschwindigkeit der Luft durch die DOR-Einrichtung durch Ändern einer Fahrzeuggeschwindigkeit oder durch Änderung einer Lüfterdrehzahl. Anschließend erfolgt im Schritt 4 eine Prüfung, ob die Einwirkung E einer Einwirkung E2 entspricht, die zur Erfassung eines Ozongehaltes C2 geeignet ist. Dazu kann beispielsweise überprüft werden, ob die Fahrzeuggeschwindigkeit innerhalb eines vorbestimmten Bereiches ist oder ob der Lüfter mit einer bestimmten Leistung angetrieben wird. Ist dies der Fall, erfolgt im Schritt 5 die Erfassung des Ozongehaltes C2 bei der Zeit T = T2. Im weiteren wird das Verfahren mit den Schritt nach Figur 1 fortgesetzt.

## Patentansprüche

1. Vorrichtung (6, 18, 20) zur Beurteilung der Funktionsfähigkeit einer in einem Kraftfahrzeug angeordneten DOR-Einrichtung (4) zur Reduzierung des Ozongehaltes von Luft,
mit einem Sensor (6) zum Erfassen des Ozongehaltes (C1, C2), der in Strömungsrichtung hinter der DOR-Einrichtung (4) angeordnet ist,
mit einer elektronischen Einrichtung (18),
die bei einer ersten Geschwindigkeit des Kraftfahrzeugs einen ersten Ozongehalt (C1) und
bei einer zweiten Geschwindigkeit einen zweiten Ozongehalt (C2) erfasst,
die eine Differenz zwischen dem ersten und zweiten Ozongehalt (C1, C2) ermittelt und
die anhand einer hinterlegten Kennlinie (OUR) die Funktionsfähigkeit der DOR-Einrichtung (4) auf der Basis der Differenz beurteilt.

2. Vorrichtung nach Anspruch 1, bei der die erste und zweite Geschwindigkeit in vorgegebenen Geschwindigkeitsbereichen liegen.

3. Vorrichtung nach Anspruch 1 oder 2, bei der die erste Geschwindigkeit des Kraftfahrzeugs der Geschwindigkeit v = 0 entspricht.

4. Vorrichtung nach einem der vorhergehenden Ansprüche, bei der die Zeitspanne (T2-Tl) zwischen der Erfassung des ersten und zweiten Ozongehalts (C1, C2) auf einen vorgegebenen maximalen Wert begrenzt ist.

5. Vorrichtung nach einem der vorhergehenden Ansprüche, dass zusätzlich ein Schwellenwert für die Motorkühlmittels-Temperatur vorgesehen ist.

6. Vorrichtung nach einem der vorhergehenden Ansprüche, bei der die DOR-Einrichtung (4) einen mit katalytisch wirkenden Oberflächenbeschichtungen (5) ausgestatteten Wärmetauscher aufweist.

## Claims

1. Device (6, 18, 20) for assessing the operational capability of a DOR device (4), arranged in a motor vehicle, for reducing the ozone content in the air, having a sensor (6) for sensing the ozone content (C1, C2), which is arranged downstream of the DOR device (4) in the direction of flow, having an electronic device (18) which senses a first ozone content (C1) when there is a first velocity of the motor vehicle and senses a second ozone content (C2) when there is a second velocity, which determines a difference between the first and the second ozone contents (C1, C2) and which assesses the operational capability of the DOR device (4) on the basis of the difference by reference to a stored characteristic curve (OUR).

2. Device according to Claim 1, in which the first and second velocities lie in predefined velocity ranges.

3. Device according to Claim 1 or 2, **characterized in that** the first velocity of the motor vehicle corresponds to the velocity of v = 0.

4. Device according to one of the preceding claims in which the time period (T2-T1) between the sensing of the first and the second ozone contents (C1, C2) is limited to a predefined maximum value.

5. Device according to one of the preceding claims, **characterized in that** in addition a threshold value for the engine coolant temperature is provided.

6. Device according to one of the preceding claims, in which the DOR device (4) has a heat exchanger which is equipped with catalytically active surface coatings (5).

## Revendications

1. Dispositif (6, 18, 20) d'évaluation de la fonctionnalité d'un dispositif DOR (4), disposé sur un véhicule automobile, pour réduire la teneur de l'air en ozone, comportant pour détecter la teneur en ozone (C1, C2), un capteur (6) disposé dans la direction de l'écoulement en arrière du dispositif DOR (4),
avec un dispositif électronique (18) qui détecte à une première vitesse du véhicule, une première teneur en ozone (C1), et à une deuxième vitesse, une deuxième teneur en ozone (C2), et
qui calcule une différence entre la première et la deuxième teneurs en ozone (C1, C2) et évalue, à l'aide d'une courbe caractéristique enregistrée (OUR), la fonctionnalité du dispositif DOR (4) sur la base de la différence.

2. Dispositif selon la revendication 1,
**caractérisé en ce que**
la première et la deuxième vitesses se situent dans des plages prédéterminées de vitesse.

3. Dispositif selon la revendication 1 ou 2,
**caractérisé en ce que**
la première vitesse du véhicule correspond à la vitesse v = 0.

4. Dispositif selon l'une des revendications précédentes,
**caractérisé en ce que**
la plage de temps (T2-T1) entre la détection de la première et de la deuxième teneurs en ozone (C1, C2) est limitée à une valeur maximale prédéterminée.

5. Dispositif selon l'une des revendications précédentes,
**caractérisé en ce qu'**
en plus, une valeur seuil de la température du réfrigérant du moteur est prévue.

6. Dispositif selon l'une des revendications précédentes,
**caractérisé en ce que**
le dispositif DOR (4) présente un échangeur thermique muni de revêtements de surface (5) à action catalytique.
